# EUROPÄISCHE PATENTSCHRIFT

(11) **EP 4 014 880 B1**
(45) Veröffentlichungstag und Bekanntmachung des Hinweises auf die Patenterteilung: **06.03.2024**
(21) Anmeldenummer: 20214651.0
(22) Anmeldetag: 16.12.2020
(51) Int. Cl.: A61C 19/04

(54) **DENTALSENSOR FÜR DEN INTRAORALBEREICH**
INTRAORAL DENTAL SENSOR
CAPTEUR DENTAIRE POUR LA ZONE INTRAORALE

(43) Veröffentlichungstag der Anmeldung: 22.06.2022
(73) Patentinhaber: Ivoclar Vivadent AG, 9494 Schaan (LI)
(72) Erfinder: NIEDRIG, Christian, 9464 Rüthi (CH); GLEBOVA, Tatjana, 9470 Buchs (CH); REINHARDT, Jonas, 7206 Igis (CH)
(74) Vertreter: Baldus, Oliver

(56) Entgegenhaltungen:
- WO-A1-2016/105592
- US-A- 5 090 047

## Beschreibung

Die vorliegende Erfindung betrifft einen Dentalsensor für einen Intraoralbereich und ein nicht therapeutisches Verfahren zum Einsetzen eines Dentalsensors.

Nicht anatomisch korrekte Sensorgehäuse für die Anwendung im Intraoralbereich bieten einen schlechten Tragekomfort, stören unnötig stark bei der Nahrungsaufnahme und sind nicht ausreichend befestigt, da die natürliche Form des Zahnbogens nicht zur zusätzlichen Haftung verwendet werden kann. Zudem besteht die Gefahr, dass sich der Dentalsensor beim Tragen ablöst und verschluckt wird.

Die Druckschrift WO 2016/105592 A1 betrifft Vorrichtungen, Kits und Verfahren zum Planen und Durchführen von chirurgischen Eingriffen oder einer Röntgenbildgebung, wie beispielsweise einer Zahnimplantat-Röntgenbildgebung oder chirurgischen Eingriffen.

Die Druckschrift US 5,090,047 A betrifft eine Vorrichtung zum Positionieren eines Bildempfängers, beispielsweise eines Röntgenfilms, im Mund eines Patienten in einer vorbestimmten Beziehung zu einem Energiestrahl, beispielsweise einem Röntgenstrahl, der außerhalb des Mundes des Patienten erzeugt wird.

Es ist die technische Aufgabe der vorliegenden Erfindung, einen Dentalsensor bereitzustellen, das auf einfache und schnelle Weise von einem Anwender im Intraoralraum befestigt werden kann.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch einen Dentalsensor für einen Intraoralbereich gelöst, mit einem Befestigungsbereich aus plastischem Material zum Abformen eines Mundbereichs beim Einsetzen des Dentalsensors, das nach dem Abformen aushärtbar ist. Das Aushärten kann innerhalb oder außerhalb der Mundhöhle erfolgen, d.h. intraoral oder extraoral. Der Mundbereich kann einen Zahnbereich mit einem oder mehreren Zähnen umfassen, der optional mit dem Zahnfleisch in Kontakt stehen kann.

Durch die Formgebung des plastischen Materials kann der Dentalsensor schnell und individuell an die räumlichen Gegebenheiten im Intraoralraum angepasst werden. Im Ergebnis kann die Position des Dentalsensors besser festgelegt werden. Die Formgebung kann beispielsweise in wenigen Minuten direkt von einem Zahnarzt vorgenommen werden. Bei einer Befestigung mittels einer anatomisch korrekten Formgebung muss nicht auf eine aufwändig zu entfernende und zahnschädigende Befestigung mittels einer chemischen Adhäsion zurückgegriffen werden.

In einer technisch vorteilhaften Ausführungsform des Dentalsensors ist das plastische Material mittels Lichts, elektromagnetischer Strahlung oder Wärme aushärtbar. Das plastische Material kann auch durch Trocknen, durch eine chemische Reaktion mit Wasser (Hydratation) oder additionsvernetzend aushärtbar sein. Dadurch wird beispielsweise der technische Vorteil erreicht, dass das Aushärten gezielt nach dem Abformen initiiert werden kann.

In einer weiteren technisch vorteilhaften Ausführungsform des Dentalsensors umfasst das plastische Material ein aushärtbares Polymer. Dadurch wird beispielsweise der technische Vorteil erreicht, dass besonders geeignete Materialien verwendet werden, die schnell aushärten. Das plastische Material kann nach dem Aushärten eine Restflexibilität oder Restelastizität behalten, so dass sich dieses leichter entfernen lässt, wie beispielsweise silikonartige Materialen. Dadurch wird beispielsweise der technische Vorteil erreicht, dass der Dentalsensor auch dann entfernt werden kann, wenn sich dieser verkeilt, beispielsweise wenn der Dentalsensor an einer Zahnlücke befestigt wird.

In einer weiteren technisch vorteilhaften Ausführungsform des Dentalsensors ist das plastische Material an einem Sensorgehäuse des Dentalsensors angeordnet. Dadurch wird beispielsweise der technische Vorteil erreicht, dass das Sensorgehäuse an dem Zahnbereich befestigt werden kann.

In einer weiteren technisch vorteilhaften Ausführungsform des Dentalsensors wird eine Verbindung zwischen dem Befestigungsbereich und dem Dentalsensor oder dem Sensorgehäuse mittels einem positiv ausgestalteten Verbindungsmittel hergestellt wird. Das positiv ausgestalteten Verbindungsmittel umfasst beispielsweise ein oder mehrere herausstehende Noppen oder Anker, die sich mit dem plastischen Material verbinden. Dabei verformt, formt oder drückt sich das plastische Material um diese Struktur. Dadurch wird beispielsweise der technische Vorteil erreicht, dass eine zuverlässige Verbindung zwischen dem Befestigungsbereich und dem Dentalsensor erzielt werden kann.

In einer weiteren technisch vorteilhaften Ausführungsform des Dentalsensors umfasst das Sensorgehäuse ein transparentes oder wärmeleitendes Material und das transparente oder wärmeleitende Material steht in Kontakt mit dem plastischen Material. Dadurch wird beispielsweise der technische Vorteil erreicht, dass das Licht oder die Wärme an das plastische Material herangeführt werden kann und das plastische Material effizient ausgehärtet werden kann.

In einer weiteren technisch vorteilhaften Ausführungsform des Dentalsensors umfasst der Dentalsensor eine Belichtungseinrichtung oder eine Heizeinrichtung für das plastische Material. Dadurch wird beispielsweise der technische Vorteil erreicht, dass das plastische Material direkt von dem Dentalsensor ausgehärtet werden kann.

In einer weiteren technisch vorteilhaften Ausführungsform des Dentalsensors ist die Belichtungseinrichtung oder die Heizeinrichtung durch einen Benutzer aktivierbar. Dadurch wird beispielsweise der technische Vorteil erreicht, dass der Benutzer die Aushärtung des plastischen Materials steuern kann.

In einer weiteren technisch vorteilhaften Ausführungsform des Dentalsensors erfolgt die Aktivierung der Belichtungseinrichtung oder der Heizeinrichtung drahtlos. Die Aktivierung der Belichtungseinrichtung oder der Heizeinrichtung kann beispielsweise über W-Lan, NFC oder Bluetooth mittels eines Mobiltelefons durchgeführt werden. Dadurch wird beispielsweise der technische Vorteil erreicht, dass keine Betätigungshandlungen im Intraoralraum erforderlich sind.

In einer weiteren technisch vorteilhaften Ausführungsform des Dentalsensors umfasst der Befestigungsbereich, der Dentalsensor und/oder das Sensorgehäuse eine vorgefertigte Durchgangsöffnung und/oder ein oder mehrere Kanäle. Dadurch wird beispielsweise der technische Vorteil erreicht, dass Flüssigkeit von dem Zahn direkt an einen Sensor geführt werden kann, der die Flüssigkeit analysiert.

In einer weiteren technisch vorteilhaften Ausführungsform des Dentalsensors ist der Bereich um die Durchgangsöffnung nicht aushärtbar. Dadurch wird beispielsweise der technische Vorteil erreicht, dass der Dentalsensor nach dem Aushärten leicht entfernt werden kann.

In einer weiteren technisch vorteilhaften Ausführungsform des Dentalsensors ist das plastische Material anatomisch vorgeformt. Die anatomische Vorformung kann beispielsweise entsprechende zahnförmige Ausbuchtungen für einzelne Zähne umfassen. Dadurch wird beispielsweise der technische Vorteil erreicht, dass sich der Dentalsensor besser an den Zahnbereich anformt.

In einer weiteren technisch vorteilhaften Ausführungsform des Dentalsensors ist auf dem plastischen Material eine Trennschicht zum Ablösen des plastischen Materials vom Zahnbereich angeordnet. Dadurch wird beispielsweise der technische Vorteil erreicht, dass der Dentalsensor nach der Abformung leicht entfernt werden kann.

Gemäß einem zweiten Aspekt wird die technische Aufgabe durch ein nicht therapeutisches Verfahren zum Einsetzen eines Dentalsensors für einen Intraoralbereich gelöst, mit den Schritten eines Abformens eines Zahnbereichs beim Einsetzen des Dentalsensors mittels eines plastischen Materials; und eines Aushärtens des plastischen Materials nach dem Abformen. Dadurch werden die gleichen technischen Vorteile wie durch das Verfahren nach dem ersten Aspekt erreicht.

In einer technisch vorteilhaften Ausführungsform des Verfahrens erfolgt das Aushärten mittels Lichts, elektromagnetischer Strahlung oder Wärme. Dadurch wird beispielsweise ebenfalls der technische Vorteil erreicht, dass das Aushärten gezielt nach dem Abformen initiiert werden kann. Ausführungsbeispiele der Erfindung sind in den Zeichnungen dargestellt und werden im Folgenden näher beschrieben.

Es zeigen:
- Fig. 1: eine schematische Seitenansicht eines Dentalsensors;
- Fig. 2: eine schematische Aufsicht auf das Dentalsensor;
- Fig. 3: eine schematische Ansicht durch den Dentalsensor;
- Fig. 4: eine schematische Ansicht durch den Dentalsensor und/oder Befestigungsbereich mit unterschiedlichen Kanälen; und
- Fig. 5: ein Blockdiagramm eines Verfahrens zum Einsetzen eines Dentalsensors.

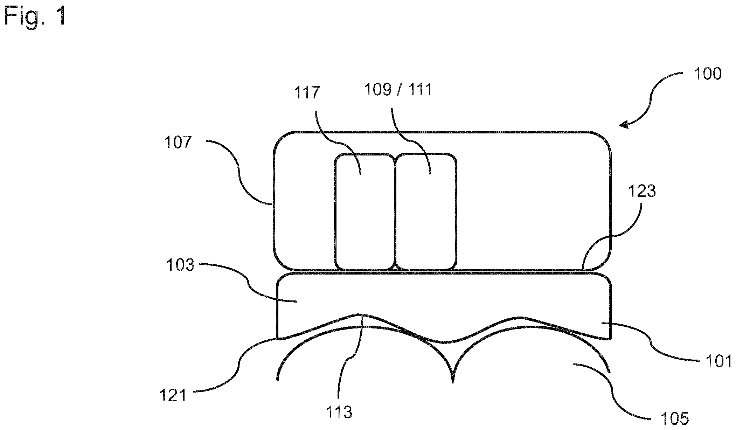

Fig. 1 zeigt eine schematische Seitenansicht eines Dentalsensors 100. Der Dentalsensor 100 umfasst ein Sensorgehäuse 107, in dem eine Auswerteelektronik 117 und eine Sensoreinheit angeordnet sind. Die Auswerteelektronik 117 und die Sensoreinheit sind zusammen geeignet, am Zahn 105 autonom Messungen von bestimmten physikalischen Parametern durchzuführen. Das Sensorgehäuse 107 ist beispielsweise in einer Standardform aus Kunststoff gefertigt.

Das Sensorgehäuse 107 weist eine ebene oder annähernd anatomisch vorgeformte zahnzugewandte Kontaktfläche 123 auf, die mit einer flächigen Schicht aus einem formbaren und plastischen Material 103 bedeckt ist. Das plastische Material 103 bildet einen Befestigungsbereich 101 zur Befestigung des Dentalsensors 100 im Mundbereich. Eine Schichtdicke des plastischen Materials 103 beträgt beispielsweise 1 mm bis 10 mm. Zur Befestigung des plastischen Materials 103 kann eine geeignete produktionsseitige Strukturierung der Kontaktfläche 123 des Sensorgehäuses 107 oder eine Verankerung des plastischen Materials 103 durch Löcher im Sensorgehäuse 107 vorgesehen sein.

Eine anatomisch geformte Kontaktfläche 123 des Sensorgehäuses 107 oder im plastischen Material 103 weist den Vorteil auf, dass sich der Tragekomfort des Dentalsensors 100 verbessert und dieser weniger bei der Nahrungsaufnahme stört. Die natürliche Form des Zahnbogens kann zur zusätzlichen Haftung des Dentalsensors 100 verwendet werden.

Das plastische und/oder verformbare Material 103 verformt sich bei einem Aufdrücken auf den Zahnbereich 105, der ein oder mehrere Zähne umfassen kann. Auf diese Weise wird durch das plastische Material 103 ein räumlicher Abdruck des Zahnbereichs 105 gewonnen. Anschließend wird das plastische Material 103 ausgehärtet, so dass dieses seine Verformbarkeit verliert. Dies kann beispielsweise mittels einer Bestrahlung mit UV-Licht, blauem Licht, im Kontakt mit Sauerstoff oder Speichel oder durch Wärme erreicht werden. Denkbar wäre ebenso die Verwendung eines Materials, das zunächst aktiviert wird und dann mit der Zeit am Zahnbereich 105 aushärtet. Vorzugsweise ist das plastische Material 103 vorgefertigt, so dass der Zeitbedarf bei der Befestigung des Dentalsensors 100 verringert wird und möglichen Fehlerquellen bei der Verarbeitung ausgeschlossen werden.

Das plastische Material 103 umfasst beispielsweise eine hydrophile Vinylpolysiloxan-Abformmasse oder ein Polymer auf Basis von Methacrylaten und verschiedenen Füllstoffen, die mit Silanen verbunden sind, wie beispielsweise ein lichthärtendes Nanohybrid-Composite.

Das plastische Material 103 umfasst beispielsweise eine Monomer-Matrix, die aus Dimethacrylates (17-18 Gew%) aufgebaut ist. Füllstoffe umfassen beispielsweise Bariumglas, Ytterbium-Trifluorid und/oder unterschiedliche Oxide und Copolymere (82-83 Gew%). Additive, Initiatoren, Stabilisatoren und Pigmente können zusätzliche Inhaltsstoffe sein (<1.0 Gew%). Die Gesamtmenge der anorganischen Füllstoffe beträgt beispielsweise zwischen 53 und 80 Vol%. Die Partikelgrößen der anorganischen Füllstoffe liegen beispielsweise zwischen 40 nm and 3 µm.

Das plastische Material 103 kann antibiotische Eigenschaften aufweisen, wie beispielsweise durch einen Einbau von Silberpartikeln, Kupferpartikeln oder eine Mischung mit Chlorhexidin and Chloroxylenol. Zudem kann das plastische Material Antibiotika umfassen, wie beispielsweise Penicillin, Clindamycin, Erythromycin, Cefadroxil, Metronidazol und/oder Tetracycline. Das plastische Material 103 kann auch durch ein Silikon oder ein Plastilin gebildet sein.

Der Dentalsensor 100 kann beispielsweise eine elektronische Belichtungseinrichtung 109 umfassen, die im Inneren des Sensorgehäuses 107 angeordnet ist. Die Belichtungseinrichtung 109 sendet Licht mittels einer Leuchtdiode aus, das zum Aushärten des lichthärtenden plastischen Materials 103 führt. In diesem Fall ist das Sensorgehäuse 107 beispielsweise aus einem optisch transparenten Material gebildet, das in Kontakt mit dem plastischen Material 103 steht. Dadurch kann das Licht zum Aushärten aus einem Inneren des Dentalsensors 100 durch das Sensorgehäuse 107 auf das plastische Material 103 treffen und dieses aushärten. Allerdings kann auch eine chemische Belichtungseinrichtung 109 vorgesehen sein, die auf dem Prinzip der Chemolumineszenz beruht, einmal aktiviert wird und chemisch erzeugtes Licht aussendet. Die chemische Belichtungseinrichtung 109 kann beispielsweise durch einen Leuchtstab gebildet sein, der in das Sensorgehäuse 107 einsetzbar ist.

Der Dentalsensor 100 kann aber auch eine Heizeinrichtung 111 aufweisen, die im Inneren des Sensorgehäuses 107 angeordnet ist. Die Heizeinrichtung 111 sendet beispielsweise Wärme mittels einer Heizspirale aus, die zum Aushärten des wärmehärtenden plastischen Materials 103 führt. In diesem Fall befindet sich zwischen der Heizeinrichtung 111 ein Metall als wärmeleitendes Material zwischen der Heizeinrichtung 111 und dem plastischen Material 103. Durch das wärmeleitende Material kann die erzeugte Wärme zum Aushärten wirksam an das plastische Material 103 geführt werden und dieses aushärten. Allerdings kann auch eine chemische Heizeinrichtung 111 vorgesehen sein, die einmal aktiviert wird und chemisch erzeugte Wärme aussendet.

Die Belichtungseinrichtung 109 oder die Heizeinrichtung 111 können manuell durch Betätigen eines Schalters oder Druckknopfes am Sensorgehäuse aktiviert werden, so dass diese für einen vorgegebenen Zeitraum leuchten oder heizen. Am Ende dieses Zeitraumes ist das plastische Material 103 ausgehärtet.

Die Belichtungseinrichtung 109 oder die Heizeinrichtung 111 können jedoch auch drahtlos über Funk aktiviert werden, beispielsweise über WLAN, NFC oder Bluetooth mittels eines Mobiltelefons oder Tablet-PCs. In diesem Fall ist in der Auswerteelektronik 117 eine entsprechende Schnittstelle implementiert, über die Belichtungseinrichtung 109 oder Heizeinrichtung 111 gesteuert werden kann.

Zudem kann eine Trennschicht 121 als Trennmittel vorgesehen sein, durch die ein Ablösen des Dentalsensors 100 von dem Zahn oder dem Zahnbereich 105 nach dem Aushärten erleichtert wird. Die Trennschicht 121 ist zusätzlich auf dem plastischen Material 103 angeordnet und verhindert einen direkten Kontakt zwischen dem Zahn 105 und dem plastischen Material 103. Diese Trennschicht 121 kann beispielsweise einen dünnen Fett- oder Ölfilm oder durch eine Schutzmembran aus Gummi, Teflon oder Latex gebildet sein. In diesem Fall kann der Dentalsensor 100 auch ohne ausgehärtetes plastisches Material 103 wieder rückstandsfrei entfernt werden.

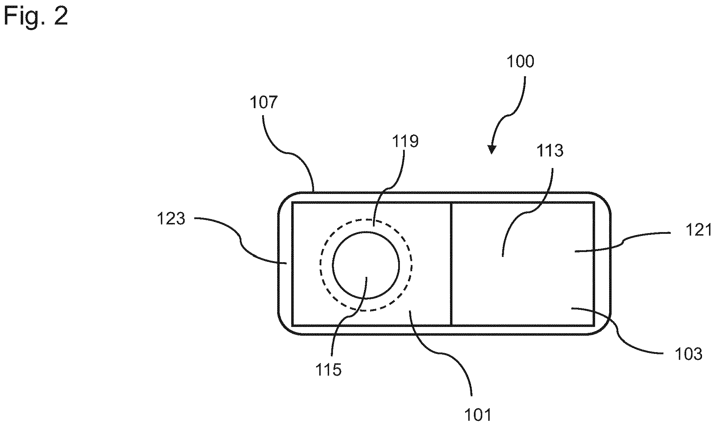

Fig. 2 zeigt eine schematische Aufsicht auf den Dentalsensor 100 und das plastische Material 103. Auf der dem Zahn 105 zugewandten Seite des plastischen Materials 103 kann bereits produktionsseitig eine Aussparung oder Durchgangsöffnung 115 in einer vorgegebenen Form vorgesehen sein, z.B. für eine verbaute Sensoreinheit oder etwaige Speichelkanäle zur besseren Zuführung von Speichel vom Zahnbereich 105 zu der Sensoreinheit im Inneren des Sensorgehäuses 107 während der intraoralen Anwendung.

Die Sensoreinheit kann beispielsweise ein Sensor zum Messen eines pH-Wertes, einer Ethanol-Konzentration, einer Lactat-Konzentration, einer Cortisol-Konzentration, einer GlucoseKonzentration, einer Ionenkonzentration, zum Messen von Schallwellen beim Aufeinanderbeißen und oder ein Sensor zum Messen einer Temperatur sein. Im Allgemeinen kann durch die Sensoreinheit innerhalb des Dentalsensors 100 eine intraorale Messung unterschiedlicher Kenngrößen über einen längeren Zeitraum durchgeführt werden

Diese vorgesehene Aussparung oder Durchgangsöffnung 115 kann beispielsweise aus plastischem Material 103 gefertigt sein, das in einem Umgebungsbereich 119 um die Durchgangsöffnung 115 herum nicht aushärtbar ist. Dies kann im Falle eines lichthärtenden Materials 103 beispielsweise dadurch geschehen, dass keine Photoinitiatoren in diesen Umgebungsbereich 119 eingebracht werden. Dieser Umgebungsbereich 119 härtet somit nach der Abformung unter Einfluss des Lichtes nicht aus und kann relativ leicht entfernt werden. Der Umgebungsbereich 119 kann auch ein wasserlösliches Material umfassen, wie beispielsweise Zucker, Maisstärke oder ein wasserlösliches Filament.

Eine anatomische Anpassung des Dentalsensors 100 kann beispielsweise durch zahnförmige oder konkave Ausbuchtungen 113 in der Kontaktfläche 123 des Dentalsensors 100 erreicht werden, die die zumindest näherungsweise dem Zahnbereich 105 entsprechen. Auf diese Weise kann der Dentalsensor 100 näher am Zahnbereich 105 angeordnet werden. Nicht nur die Kontaktfläche 123, sondern auch das plastische Material 103 kann mit entsprechenden zahnförmigen oder konkaven Ausbuchtungen 113 anatomisch vorgeformt sein.

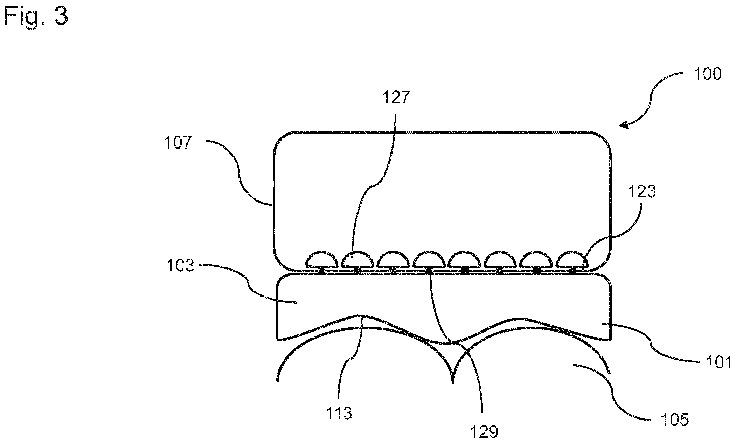

Fig. 3 zeigt eine weitere schematische Ansicht durch den Dentalsensor 100. Die Befestigung des Befestigungsbereichs 101 mit dem plastischen Material 103 erfolgt durch eine Mehrzahl von Öffnungen 129, die in dem Sensorgehäuse 107 angeordnet sind. Das plastische und ungehärtete Material 103 wird beim Befestigen durch die Öffnungen 129 in der Gehäusewand teilweise hindurchgedrückt und anschließend auf der Innenseite des Sensorgehäuse 107 plattgedrückt. Danach kann ein innenseitiger Aushärteschritt erfolgen, um die platten Strukturen 127 zu verfestigen. Auf diese Weise krallt sich das plastischen Material 103 am Sensorgehäuse 107 fest und kann sich nicht mehr lösen. Das plastische Material 101 hält sich selbst durch die pilzförmigen Strukturen 127 nach dem Aushärten.

Eine andere Möglichkeit der Befestigung kann durch eine Mikrostrukturierung der Kontaktfläche 123 erreicht werden, wie beispielsweise durch einfaches Anschleifen.

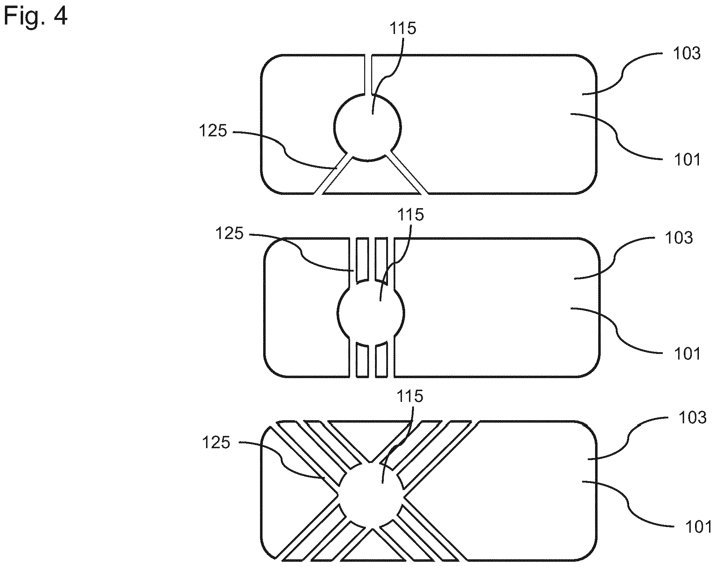

Fig. 4 zeigt eine schematische Ansicht des Befestigungsbereiches 101, des Dentalsensors 100 und/oder des Dentalsensorgehäuses 107 mit unterschiedlichen Kanälen 125. Des Weiteren kann in dem plastischen Material 103 die Durchgangsöffnung 115 zum Ermöglichen einer Messung durch Sensoreinheit vorgesehen sein. Die Durchgangsöffnung 115 bildet einen zusätzlichen Messbereich für die Sensoreinheit neben den Kanälen. Die Durchgangsöffnung 115 kann beispielsweise aus plastischem Material 103 gefertigt sein, das in einem Umgebungsbereich 119 um die Durchgangsöffnung 115 herum nicht aushärtbar ist.

Die Kanäle 125 sind in der dem plastischen Material 103 durch Vertiefungen gebildet und dienen dazu Flüssigkeit (Speichel) zu einer Sensoreinheit zu leiten oder durchzuführen oder einen Luftaustausch und eine Ventilation des Messbereichs zu ermöglichen. Die Kanäle 125 können in horizontaler, diagonaler oder vertikaler Richtung angeordnet sein.

Die Kanäle 125 können beispielsweise dadurch gebildet werden, dass an den vorgesehenen Stellen der Kanäle 125 kein Photoinitiator in das lichthärtende plastischen Material 103 eingebracht wird. Nach einem Aushärten mittels Lichts kann an diesen Stellen das nicht-härtbare plastische Material 103 ohne die Photoinitiatoren entfernt werden, wie beispielsweise mit einem Wasserstrahl oder einem Spatel, um so die Kanäle 125 in dem Befestigungsbereich 101 zu erhalten.

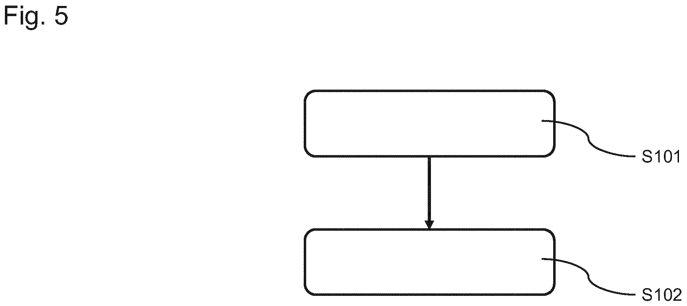

Fig. 5 zeigt ein Blockdiagramm eines Verfahrens zum Einsetzen eines Dentalsensors 100. Im Schritt S101 wird der Zahnbereich 105 beim Einsetzen des Dentalsensors 100 mittels eines plastischen Materials 103 abgeformt, indem dieses auf den Zahnbereich gedrückt wird. Das plastische Material 103 passt sich dabei der Form des Zahns 105 an. Anschließend wird im Schritt S102 das plastische Material 103 nach dem Abformen ausgehärtet. Je nach Ausgestaltung kann der Dentalsensor 100 dabei in der Mundhöhle verbleiben oder außerhalb der Mundhöhle ausgehärtet werden. Auf diese Weise kann der Befestigungsbereich 101 an einem Zahn 105 befestigt werden.

Die individuelle Formgebung kann in wenigen zeitsparenden Arbeitsschritten direkt im Mund eines Patienten in nur einer Sitzung bewerkstelligt werden. Durch eine derartig anatomisch individuelle Formgebung eines intraoralen Sensorträgers auf den Patienten ist der Dentalsensor 100 geeignet, dauerhaft getragen zu werden. Dieser kann bei einer Nahrungsaufnahme, bei Gesprächen und im Schlaf getragen werden. Im Vergleich zum individuellen 3D-Druck mittels Abformung, Scan und Produktion im Zahnlabor kann die Formgebung und Anpassung des Dentalsensors 100 in kurzer Zeit direkt vom Zahnarzt vorgenommen werden. Daneben wird eine Sensorpositionierung nahe am Zahn ermöglicht, da der Dentalsensor 100 anatomisch individuell auf den Patienten angepasst ist.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Einrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentalsensor
- 101: Befestigungsbereich
- 103: plastisches Material
- 105: Zahnbereich/Zahn
- 107: Sensorgehäuse
- 109: Belichtungseinrichtung
- 111: Heizeinrichtung
- 113: Ausbuchtung
- 115: Durchgangsöffnung
- 117: Auswertelektronik
- 119: Umgebungsbereich
- 121: Trennschicht
- 123: Kontaktfläche
- 125: Kanal
- 127: Struktur
- 129: Öffnung

## Patentansprüche

1. Dentalsensor (100) für einen Intraoralbereich, mit:
einem Befestigungsbereich (101) aus plastischem Material (103) zum Abformen eines Mundbereichs (105) beim Einsetzen des Dentalsensors (100), das nach dem Abformen aushärtbar ist; und
einem Sensorgehäuse (107), das eine ebene oder annähernd anatomisch vorgeformte zahnzugewandte Kontaktfläche (123) aufweist, die mit einer flächigen Schicht aus dem formbaren und plastischen Material (103) bedeckt ist.

2. Dentalsensor (100) nach Anspruch 1, wobei das plastische Material (103) mittels Lichts, elektromagnetischer Strahlung oder Wärme aushärtbar ist

3. Dentalsensor (100) nach einem der vorangehenden Ansprüche, wobei das plastische Material (103) ein aushärtbares Polymer umfasst.

4. Dentalsensor (100) nach einem der vorangehenden Ansprüche, wobei das plastische Material (103) an einem Sensorgehäuse (107) des Dentalsensors (100) angeordnet ist.

5. Dentalsensor (100) nach einem der vorangehenden Ansprüche, wobei eine Verbindung zwischen dem Befestigungsbereich (101) und dem Dentalsensor (100) oder dem Sensorgehäuse (107) mittels einem positiv ausgestalteten Verbindungsmittel hergestellt wird.

6. Dentalsensor (100) nach Anspruch 4, wobei das Sensorgehäuse (107) ein transparentes oder wärmeleitendes Material umfasst und das transparente oder wärmeleitende Material in Kontakt mit dem plastischen Material steht.

7. Dentalsensor (100) nach einem der vorangehenden Ansprüche, wobei der Dentalsensor (100) eine Belichtungseinrichtung (109) oder eine Heizeinrichtung (111) für das plastische Material umfasst.

8. Dentalsensor (100) nach Anspruch 7, wobei die Belichtungseinrichtung (109) oder die Heizeinrichtung (111) durch einen Benutzer aktivierbar ist.

9. Dentalsensor (100) nach Anspruch 8, wobei die Aktivierung der Belichtungseinrichtung (109) oder der Heizeinrichtung (111) drahtlos erfolgt.

10. Dentalsensor (100) nach einem der vorangehenden Ansprüche, wobei der Befestigungsbereich (101), der Dentalsensor (100) und/oder das Sensorgehäuse (107) eine vorgefertigte Durchgangsöffnung (115) und/oder ein oder mehrere Kanäle (125) umfasst.

11. Dentalsensor (100) nach einem der vorangehenden Ansprüche, wobei der Umgebungsbereich (119) des plastischen Materials (103) um die Durchgangsöffnung (115) nicht aushärtbar ist.

12. Dentalsensor (100) nach einem der vorangehenden Ansprüche, wobei das plastische Material (103) anatomisch vorgeformt ist.

13. Dentalsensor (100) nach einem der vorangehenden Ansprüche, wobei auf dem plastischen Material (103) eine Trennschicht (121) zum Ablösen des plastischen Materials (103) vom Mundbereich (103) angeordnet ist.

14. Nicht therapeutisches Verfahren zum Einsetzen eines Dentalsensors (100) für einen Intraoralbereich, mit den Schritten:
- Abformen (S101) eines Mundbereichs (105) beim Einsetzen des Dentalsensors (100) mittels eines plastischen Materials (103) auf einem Sensorgehäuse (107), das eine ebene oder annähernd anatomisch vorgeformte zahnzugewandte Kontaktfläche (123) aufweist, die mit einer flächigen Schicht aus dem formbaren und plastischen Material (103) bedeckt ist; und
- Aushärten (S102) des plastischen Materials (103) nach dem Abformen.

15. Verfahren nach Anspruch 14, wobei das Aushärten mittels Lichts, elektromagnetischer Strahlung oder Wärme erfolgt.

## Claims

1. A dental sensor (100) for an intraoral region, comprising:
an attachment region (101) of plastic material (103) for molding an oral region (105) during insertion of the dental sensor (100), which is curable after molding; and
a sensor housing (107) which has a plane or approximately anatomically preformed contact surface (123) facing the tooth which is covered with a flat layer of the formable and plastic material (103).

2. The dental sensor (100) according to claim 1, wherein the plastic material (103) is curable by means of light, electromagnetic radiation, or heat.

3. The dental sensor (100) according to any one of the preceding claims, wherein the plastic material (103) comprises a curable polymer.

4. The dental sensor (100) according to any one of the preceding claims, wherein the plastic material (103) is arranged on a sensor housing (107) of the dental sensor (100).

5. The dental sensor (100) according to any one of the preceding claims, wherein a connection between the attachment region (101) and the dental sensor (100) or the sensor housing (107) is established by means of a positively configured connecting means.

6. The dental sensor (100) according to claim 4, wherein the sensor housing (107) comprises a transparent or thermally conductive material and the transparent or thermally conductive material is in contact with the plastic material.

7. The dental sensor (100) according to any one of the preceding claims, wherein the dental sensor (100) comprises a light exposure device (109) or a heating device (111) for the plastic material.

8. The dental sensor (100) according to claim 7, wherein the light exposure device (109) or the heating device (111) is activatable by a user.

9. The dental sensor (100) according to claim 8, wherein the activation of the light exposure device (109) or the heating device (111) is wireless.

10. The dental sensor (100) according to any one of the preceding claims, wherein the attachment region (101), the dental sensor (100) and/or the sensor housing (107) comprises a prefabricated through opening (115) and/or one or more channels (125).

11. The dental sensor (100) according to any one of the preceding claims, wherein the surrounding region (119) of the plastic material (103) around the through opening (115) is not curable.

12. The dental sensor (100) according to any one of the preceding claims, wherein the plastic material (103) is anatomically preformed.

13. The dental sensor (100) according to any one of the preceding claims, wherein a separation layer (121) is arranged on the plastic material (103) for removing the plastic material (103) from the oral region (103).

14. A non-therapeutic method of inserting a dental sensor (100) for an intraoral region, comprising the steps of:
- molding (S101) an oral region (105) during insertion of the dental sensor (100) by means of a plastic material (103) on a sensor housing (107) which has a plane or approximately anatomically preformed contact surface (123) facing the tooth which is covered with a flat layer of the formable and plastic material (103); and
- curing (S102) the plastic material (103) after the molding.

15. The method according to claim 14, wherein the curing is performed by means of light, electromagnetic radiation, or heat.

## Revendications

1. Capteur dentaire (100) pour une zone intra-buccale, avec :
- une zone de fixation (101) en matière plastique (103) pour la prise d'empreinte d'une zone buccale (105) lors de l'insertion du capteur dentaire (100), qui peut être durcie après la prise d'empreinte ; et
- un boîtier de capteur (107) qui présente une surface de contact (123) plane ou préformée de manière approximativement anatomique, orientée vers les dents, recouverte d'une couche plate de matière malléable et plastique (103).

2. Capteur dentaire (100) selon la revendication 1, où la matière plastique (103) peut être durcie au moyen de la lumière, d'un rayonnement électromagnétique ou de la chaleur.

3. Capteur dentaire (100) selon l'une des revendications précédentes, où la matière plastique (103) est constituée d'un polymère durcissable.

4. Capteur dentaire (100) selon l'une des revendications précédentes, où la matière plastique (103) est disposée sur un boîtier de capteur (107) du capteur dentaire (100).

5. Capteur dentaire (100) selon l'une des revendications précédentes, où une connexion entre la zone de montage (101) et le capteur dentaire (100) ou le boîtier du capteur (107) est établie au moyen d'un moyen de connexion de conception positive.

6. Capteur dentaire (100) selon la revendication 4, où le boîtier du capteur (107) est constitué d'un matériau transparent ou thermoconducteur et le matériau transparent ou thermoconducteur est en contact avec la matière plastique.

7. Capteur dentaire (100) selon l'une des revendications précédentes, où le capteur dentaire (100) comprend un dispositif d'exposition (109) ou un dispositif de chauffage (111) pour la matière plastique.

8. Capteur dentaire (100) selon la revendication 7, où le dispositif d'exposition (109) ou le dispositif de chauffage (111) peut être activé par un utilisateur.

9. Capteur dentaire (100) selon la revendication 8, où l'activation du dispositif d'exposition (109) ou du dispositif de chauffage (111) est réalisée sans fil.

10. Capteur dentaire (100) selon l'une des revendications précédentes, où la zone de montage (101), la capteur dentaire (100) et/ou le boîtier de capteur (107) comprennent une ouverture de passage préfabriquée (115) et/ou un ou plusieurs conduits (125).

11. Capteur dentaire (100) selon l'une des revendications précédentes, où la zone environnante (119) de la matière plastique (103) autour de l'ouverture du passage (115) n'est pas durcissable.

12. Capteur dentaire (100) selon l'une des revendications précédentes, où la matière plastique (103) est préformée de manière anatomique.

13. Capteur dentaire (100) selon l'une des revendications précédentes, où une couche de séparation (121) est disposée sur la matière plastique (103) pour détacher la matière plastique (103) de la région buccale (103).

14. Procédure non thérapeutique pour l'insertion d'un capteur dentaire (100) pour une zone intra-buccale, comprenant les étapes suivantes :
- empreinte (Sl01) d'une zone buccale (105) lors de l'insertion du capteur dentaire (100) au moyen d'une matière plastique (103) sur un boîtier de capteur (107) qui présente une surface de contact (123) plane ou préformée de manière approximativement anatomique orientée vers les dents recouverte d'une couche plane de matière malléable et plastique (103) ; et
- durcissement (S102) de la matière plastique (103) après le moulage.

15. Procédé selon la revendication 14, où le durcissement est effectué au moyen de la lumière, d'un rayonnement électromagnétique ou de la chaleur.
